# EUROPÄISCHE PATENTSCHRIFT

(11) **EP 2 600 917 B1**
(45) Veröffentlichungstag und Bekanntmachung des Hinweises auf die Patenterteilung: **31.10.2018**
(21) Anmeldenummer: 11740575.3
(22) Anmeldetag: 01.08.2011
(51) Int. Cl.: A61M 1/10, F04B 43/12

(54) **VORRICHTUNG MIT EINER SCHLAUCHROLLENPUMPE**
DEVICE WITH A PERISTALTIC HOSE PUMP
DISPOSITIF MUNI D'UNE POMPE PÉRISTALTIQUE

(30) Priorität: 03.08.2010 DE 102010033241
(43) Veröffentlichungstag der Anmeldung: 12.06.2013
(73) Patentinhaber: Fresenius Medical Care Deutschland GmbH, 61352 Bad Homburg (DE)
(72) Erfinder: GRONAU, Sören, 64569 Nauheim (DE); MÜLLER, Ralf, 61348 Bad Homburg (DE)
(74) Vertreter: Herrmann, Uwe
(86) Internationale Anmeldenummer: PCT/EP2011/003850
(87) Internationale Veröffentlichungsnummer: WO 2012/016677

(56) Entgegenhaltungen:
- WO-A1-2011/080187
- US-A- 5 215 450
- US-A1- 2005 209 552

## Beschreibung

Die vorliegende Erfindung betrifft eine Vorrichtung mit einer Schlauchrollenpumpe.

In der Dialyse werden im Allgemeinen Schlauchrollenpumpen zur Förderung von Flüssigkeiten wie Substituat oder Blut eingesetzt. Die Rollen dieser Peristaltikpumpen sind federgelagert, um den im Fehlerfall aufgebauten maximalen Druck zu begrenzen. Im normalen Betrieb wird davon ausgegangen, dass während des Eingriffs einer Rolle vollständige Okklusion herrscht. Ob dies der Fall ist oder nicht unterliegt keiner ständigen Überwachung. Die Funktion des Rotors wird lediglich werkseitig geprüft.

Im Verlauf der Dialyse steigt typischerweise der Vorfilterdruck bzw. Prefilterdruck (Druck zwischen Blutpumpe und Dialysator) an. Der Flusswiderstand des Dialysators erhöht sich z. B. aufgrund des Verschlusses einzelner Kapillaren. Normalerweise ist der Fluss von Schlauchrollenpumpen nicht gegendruckabhängig. Übersteigt der Gegendruck jedoch einen Grenzwert, kann die sich aktuell im Eingriff befindliche Rolle kurzfristig abheben. Ein Verlust der Okklusion führt zur Reduzierung des tatsächlichen Blutflusses, viel wichtiger ist aber, dass ein Rotor in diesem Zustand zu erhöhter Hämolyse führen kann.

Aus dem Stand der Technik sind bereits Ansätze bekannt, das Arbeitsverhalten einer Schlauchrollenpumpe zu überwachen, sowie auch Ansätze, um Stenosen im Zusammenhang mit einem extrakorporalen Blutkreislauf zu erkennen.

Die DE 10 2006 011 346 A1 betrifft ein Verfahren und eine Vorrichtung zum Betreiben einer elektrischen peristaltischen Schlauchpumpe, wobei mittels einer Auswertung der elektrischen Leistungsaufnahme der Schlauchrollenpumpe überprüft wird, ob eine vollständige oder eine nur noch teilweise Okklusion der Schlauchrollenpumpe vorliegt.

Die EP 1 245 242 A2 betrifft ein Verfahren und eine Vorrichtung zum Erkennen von Stenosen in einem Schlauchleitungssystem, wobei das Schlauchleitungssystem eine Schlauchrollenpumpe zur Förderung eines Fluids aufweist. Hierbei wird der oszillierende Druck flussaufwärts der Pumpe ausgewertet und aufgrund einer Veränderung des Drucksignals auf eine Stenose geschlossen wird.

Die DE 199 01 078 C1 beschreibt eine Vorrichtung zur Erkennung von Stenosen bei der extrakorporalen Blutbehandlung. Hierzu wird das mittels des arteriellen Drucksensors aufgenommene Drucksignal analysiert. Hierbei wird durch die Druckpumpe ein oszillierendes Drucksignal generiert, dass sich über das Schlauchleitungssystem fortpflanzt. Bei einer hinreichend starken Stenose zwischen Blutpumpe und Blutbehandlungseinheit steigt der Druck nach der Blutpumpe so stark an, dass die Infusion der Pumpenrollen teilweise aufgehoben wird. Dies hat zur Folge, dass beim Pumpvorgang zeitweise etwas Blut in das Leitungssystem vor die Blutpumpe strömt, was zu einer Vergrößerung der am arteriellen Drucksensor gemessenen Pulsamplitude führt. Gleichzeitig bewegt sich der im Allgemeinen negative arterielle Mitteldruck in Richtung der Nullinie, da die Fördermenge nachlässt. Entsprechend kann auf eine Stenose geschlossen werden.

Aus der WO 97/10013 ist ein Verfahren zur Erkennung einer Stenose am Patientenzugang bekannt, bei dem ein oszillierendes Drucksignal analysiert wird, das über das Schlauchleitungssystem übertragen wird. Aus dem Drucksignal wird das auf die Rotation der Blutpumpe zurückzuführende Pumpensignal extrahiert, um das Pulssignal des Patienten detektieren zu können. Sofern das Pulssignal des Patienten nicht detektierbar ist, wird auf eine Stenose im extrakorporalen Blutkreislauf bzw. im Anschluss des extrakorporalen Blutkreislaufs geschlossen.

Schließlich zeigt die WO 2011/080187 A1 eine Vorrichtung gemäß dem Oberbegriff des Anspruchs 1.

Aufgabe der vorliegenden Erfindung ist es, eine Vorrichtung der eingangs genannten Art in vorteilhafter Weise weiterzubilden, insbesondere dahingehend, dass eine mögliche Blutschädigung durch einen nicht vollständig okkludierenden Rotor einer Schlauchrollenpumpe möglichst umgehend erkennbar und eine Verbesserung der Betriebssicherheit einer Schlauchrollenpumpe erreicht werden kann.

Diese Aufgabe wird erfindungsgemäß gelöst durch eine Vorrichtung mit den Merkmalen des Anspruchs 1. Danach ist vorgesehen, dass eine Vorrichtung mit wenigstens einer Schlauchrollenpumpe, wobei die Schlauchrollenpumpe wenigstens ein Pumpenbett und wenigstens einen in das Pumpenbett eingelegten oder einlegbaren Schlauch oder Schlauchteil aufweist, und mit wenigstens einem stromabwärts der Schlauchrollenpumpe angeordneten Druckerfassungsmittel, das so konfiguriert ist, dass es den zeitlichen Verlauf des Druckes eines mittels der Schlauchrollenpumpe geförderten Fluids ermittelt, und mit wenigstens einem Überwachungsmittel versehen ist, das so konfiguriert ist, dass es die Okklusion des in das Pumpenbett eingelegten oder einlegbaren Schlauches oder Schlauchteils anhand der Zeit- und/oder der Winkeldifferenz zwischen wenigstens einem Maximum und wenigstens einem Minimum des ermittelten zeitlichen Verlaufs des Druckes und/oder anhand eines lokalen Minimums des ermittelten zeitlichen Verlaufes des Druckes überwacht, wobei in einem Fall, in dem eine verringerte Okklusion erkannt wird, eine Reduzierung der Förderrate zur oder bis zur Wiederherstellung einer vollständigen Okklusion automatisch oder halbautomatisch vorgenommen wird.

Bei der Winkeldifferenz handelt es sich insbesondere um die Differenz der Winkellagen des Rotors der Schlauchrollenpumpe zwischen den Maxima und Minima des Drucks. Zeit- und Winkeldifferenz können miteinander korrelieren, da beide von der Drehbewegung des Rotors abhängen können.

Das Überwachungsmittel kann beispielsweise durch eine Steuerungs- und/oder Regelungseinheit wie z. B. durch einen entsprechenden Controller oder Mikrocomputer ausgebildet sein. Grundsätzlich ist auch denkbar, dass in einem Fall, in dem die Vorrichtung mit einer größeren bzw. anderen Maschine zusammenwirkt, die Funktion des Überwachungsmittels durch einen Teil der Maschinensteuerung der Maschine übernommen wird.

Besonders vorteilhaft ist, dass nunmehr anhand des zeitlichen Verlaufs der Druckmaxima gegenüber den Druckminima eine besonders einfache Überwachung der Okklusion einer Schlauchrollenpumpe möglich ist. Somit kann eine mögliche Blutschädigung durch einen nicht vollständig okkludierenden Rotor der Schlauchrollenpumpe schnell und sicher erkannt werden. Auch ein mechanischer Defekt des Rotors, welcher sich z. B. durch eine erhöhte Reibung der Schwingung oder durch einen Rollenschiefstand auszeichnet, kann erkannt werden. Denn auch ein derartiger mechanischer Defekt hat Auswirkungen auf den zeitlichen Verlauf des Druckes und kann dementsprechend detektiert werden.

Von der Erfindung ist auch der Fall umfasst, dass eine verringerte Okklusion dadurch erkannt wird, dass der zeitliche Verlauf des Druckes eines mittels der Schlauchrollenpumpe geförderten Fluids ein lokales Minimum aufweist.

Darüber hinaus kommt es zu einem Sicherheitszuwachs, da die Pumpfunktion direkt überwacht werden kann und keine Einbeziehung der schlecht berechenbaren und zeitabhängigen Eigenschaften eines disposablen Materials z. B. eines Schlauches mehr notwendig ist, wie dies beispielsweise bei Verfahren der Fall ist, die ein Drucksignal auswerten, das durch das eingesetzte Schlauchmaterial beeinflusst wird.

Es kann vorgesehen sein, dass das Druckerfassungsmittel stromabwärts der Schlauchrollenpumpe und stromaufwärts eines Filters, insbesondere eines Dialysators, angeordnet ist, und der Druckverlauf in diesem Bereich ein Prefilterdruckverlauf ist, wobei der stromabwärts der Schlauchrollenpumpe erfaßte zeitliche Verlauf des Druckes der Prefilterdruckverlauf ist und dass der Prefilterdruckverlauf kontinuierlich erfassbar ist.

Es kann des Weiteren vorgesehen sein, dass anhand eines Ansteigens des zeitlichen Abstandes zwischen Maximum und Minimum des zeitlichen Verlaufs des Druckes eine verringerte Okklusion erkennbar ist, insbesondere ein vorzeitiges Abheben einer Rolle der Schlauchrollenpumpe erkannt wird.

Es ist möglich, dass die Vorrichtung wenigstens ein Warnmittel aufweist und dass in einem Fall, in dem eine verringerte Okklusion erkannt wird, mittels des Warnmittels wenigstens eine Warnmeldung abgebbar ist.

Darüber hinaus kann es vorgesehen sein, dass die Überwachung des zeitlichen Verlaufs des Druckes zusammen mit einem Abgleich der Winkelposition des Schlauchrollenrotors der Schlauchrollenpumpe durchführbar ist.

Es ist ferner möglich, dass die Vorrichtung Bestandteil einer Blutbehandlungsvorrichtung ist und/oder mit einer Blutbehandlungsvorrichtung in Verbindung steht und/oder eine Blutbehandlungsvorrichtung ist und/oder umfasst.

Denkbar ist auch, dass zumindest Teile der Vorrichtung als Disposable ausgeführt sind, insbesondere in eine disposable Kassette zum Einsatz in eine derartige Blutbehandlungsvorrichtung integriert sind. Insbesondere kann in einer solchen Kassette zumindest ein Schlauchteil zum Einsatz in die Schlauchrollenpumpe und/oder das Druckerfassungsmittel oder zumindest ein Teil des Druckerfassungsmittels angeordnet sein.

In weiterer Ausgestaltung der Erfindung umfasst die Vorrichtung Mittel, mittels derer die Winkelposition des Rotors der Schlauchrollenpumpe ermittelt werden kann.

Die Winkelposition des Schlauchrollenrotors der Schlauchrollenpumpe kann beispielsweise über Hall-Sensoren, optische Sensoren, Drehwinkelpotentiometer, etc. detektiert und dem Überwachungsmittel übermittelt werden.

Die erfindungsgemäße Vorrichtung eignet sich zur Durchführung eines Verfahrens zur Überwachung der Okklusion einer Schlauchrollenpumpe, das derart durchgeführt wird, dass die Schlauchrollenpumpe wenigstens ein Pumpenbett und wenigstens einen in das Pumpenbett eingelegten oder einlegbaren Schlauch oder Schlauchteil aufweist, dass stromabwärts der Schlauchrollenpumpe der zeitliche Verlauf des Druckes eines mittels der Schlauchrollenpumpe geförderten Fluids ermittelt wird und die Okklusion des in das Pumpenbett eingelegten oder einlegbaren Schlauches oder Schlauchteils anhand der Zeit- und/oder der Winkeldifferenz zwischen wenigstens einem Maximum und wenigstens einem Minimum des ermittelten zeitlichen Verlaufs des Druckes und/oder anhand eines lokalen Minimums des ermittelten zeitlichen Verlaufes des Druckes überwacht wird.

Darüber hinaus kann vorgesehen sein, dass der stromabwärts der Schlauchrollenpumpe erfasste zeitliche Verlauf des Druckes der Prefilterdruckverlauf ist und dass der Prefilterdruckverlauf kontinuierlich erfasst wird.

Ferner ist denkbar, dass anhand eines Ansteigens des zeitlichen Abstandes zwischen Maximum und Minimum des zeitlichen Verlaufs des Druckes eine verringerte Okklusion erkannt wird, insbesondere ein vorzeitiges Abheben einer Rolle der Schlauchrollenpumpe erkannt wird. Insbesondere ist ein Abheben der Schlauchrolle vom Schlauch aufgrund des hohen Gegendrucks flussabwärts gleichbedeutend mit einer nicht mehr vorhandenen Okklusion zwischen dem Schlauchsegment im Pumpenbett nach der okkludierenden Rolle, die zum Eingang okkludiert, und dem Ausgang der Pumpe. Es erfolgt somit eine plötzliche Entspannung des Drucks flussabwärts der Schlauchrollenpumpe, was zur Erkennung der Veränderung der Okklusion herangezogen werden kann. Im Normalbetrieb, also ohne vorzeitiges Abheben der Rolle, erfolgt diese Entspannung immer in gleicher Position der Rollen, nämlich dann, wenn die eine Rolle das Pumpenbett nach unten verlässt und den eingelegten Schlauch nicht mehr okkludieren kann. Kurz vor diesem Zeitpunkt oder der entsprechenden Winkelstellung des Rotors der Schlauchrollenpumpe ist der Druck flussabwärts der Rollenpumpe maximal, kurz danach ist er minimal. Ein vorzeitiges Abheben der Rolle durch zu hohen Gegendruck kann somit durch eine verfrühte Entspannung des Nachpumpendrucks erkannt werden.

Weiterhin ist denkbar, dass eine verringerte Okklusion dadurch erkannt wird, dass der Druckverlauf ein lokales Minimum aufweist. Es hat sich in der Praxis gezeigt, dass vor dem Verlust der Okklusion der Prefilterdruck ein lokales Minimum ausbilden kann. Ein lokales Minimum ist hierbei dadurch gekennzeichnet, dass der Prefilterdruck im zeitlichen Verlauf zunächst fällt, um kurze Zeit später wieder anzusteigen, ohne, dass der Druckwert ein absolutes Minimum annimmt. Die Detektion eines solchen lokalen Minimums kann zur Erkennung einer verringerten Okklusion herangezogen werden.

In einem Fall, in dem eine verringerte Okklusion erkannt wird, kann wenigstens eine Warnmeldung abgegeben werden, und wird eine Reduzierung der Förderrate zur oder bis zur Wiederherstellung einer vollständigen Okklusion automatisch oder halbautomatisch vorgenommen. Weiter ist es möglich, dass die Überwachung des zeitlichen Verlaufs des Druckes zusammen mit einem Abgleich der Winkelposition des Schlauchrollenrotors der Schlauchrollenpumpe erfolgt.

Wie oben ausgeführt, können geeignete Mittel vorgesehen sein, mittels derer die Winkelposition des Schlauchrollenrotors erfaßt wird. Diese Mittel können beispielsweise Hall-Sensoren, optische Sensoren, Drehwinkelpotentiometer, etc. erfassen. Vorzugsweise sind diese Mittel so ausgeführt, dass sie die erfaßte Winkelposition in Form eines Signals an die Überwachungsmittel übertragen oder mit solchen Übertragungsmitteln in Verbindung stehen.

Weiterhin betrifft die vorliegende Erfindung eine Blutbehandlungsvorrichtung mit den Merkmalen des Anspruchs 9. Danach ist vorgesehen, dass eine Blutbehandlungsvorrichtung, insbesondere eine Hämodialysemaschine, mit wenigstens einer erfindungsgemäßen Vorrichtung versehen ist.

Weitere Einzelheiten und Vorteile der Erfindung sollen nun anhand eines in der Zeichnung dargestellten Ausführungsbeispiels näher läutert werden. Es zeigen:
- Figur 1:: eine schematische Betrachtung der anliegenden Kräfte und Drücke in einer Schlauchrollenpumpe;
- Figur 2:: ein Diagramm betreffend die Kraft-/Weg-Kennlinien von Schlauchmaterialien;
- Figur 3:: die Federkennlinie eines Rotors einer Schlauchrollenpumpe; und
- Figur 4:: ein Diagramm betreffend den Prefilterdruckverlauf mit unterschiedlichen Kennlinien bei zunehmendem Gegendruck.

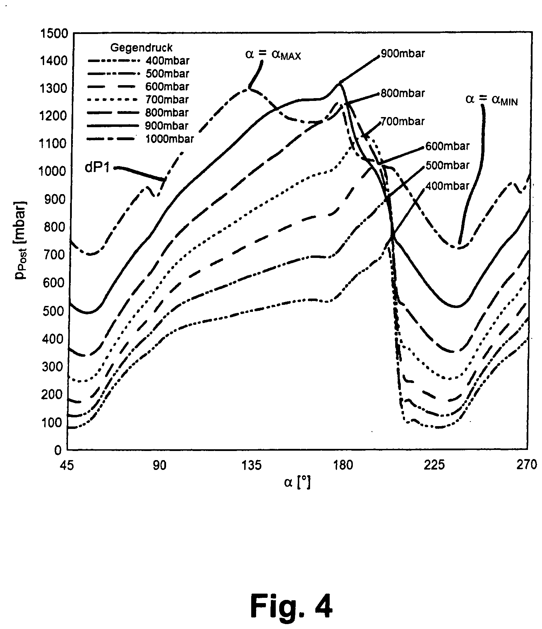

Das Verfahren wird vorteilhafterweise auf einer nicht näher dargestellten Vorrichtung folgendermaßen durchgeführt:
Das Prefilterdrucksignal wird in möglichst hoher zeitlicher Auflösung erfasst. Der Verlauf des Drucksignals im Arbeitsbereich ist weitgehend reproduzierbar, d. h. bei gleichem Drehwinkel α (vgl. auch Figur 4) kann in der Regel auch der gleiche Druck gemessen werden.

Die Form des Druckverlaufs wird vor allem durch das Pumpprinzip bestimmt. Am Prefilterdrucksensor ist das Fördern (Anstieg des Drucks bzw. Druck konstant) und das Ausgreifen der Rolle (abrupter Druckabfall) im Druckverlauf charakteristisch erkennbar.

Die Winkelposition des Abhebens der Rolle ist abhängig von der Form des Pumpbetts und von der (resultierenden) Kraft, mit welcher der Schlauch zusammengedrückt wird.

Das Pumpbett ist so geformt, dass der Rollenausgriff ab einem definierten Winkel eingeleitet wird. Die Rolle folgt der Auslaufschräge solange, bis sie ihre Endposition erreicht hat und die Schwinge am inneren Anschlag des Rotors am weiteren Ausfahren gehindert wird. Die Kraft resultiert zum einen aus der Federkraft mit der die Rolle gegen den Schlauch drückt und zum anderen aus dem Druck im Schlauch der dieser Federkraft entgegenwirkt sowie der Rückstellkraft des Schlauchmaterials.

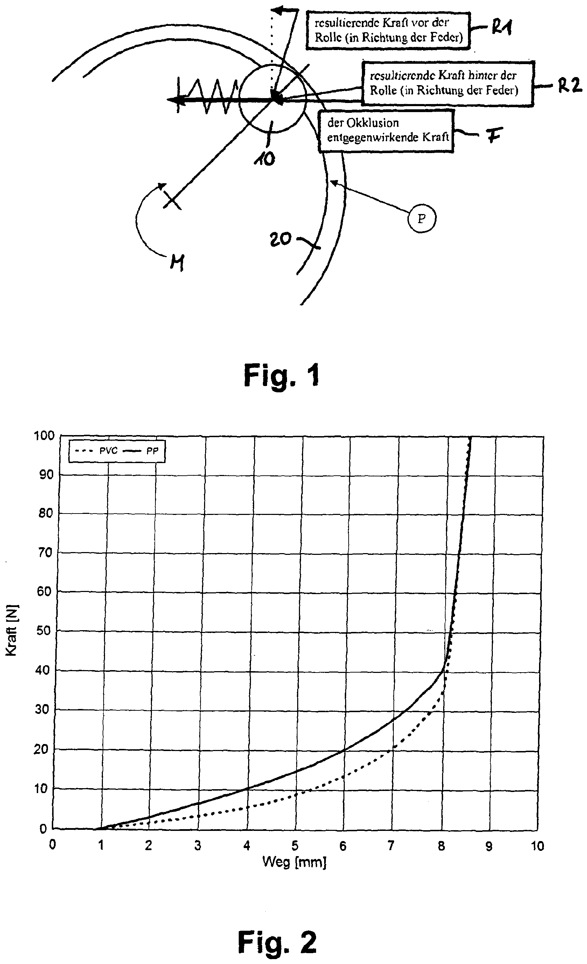

Figur 1 zeigt schematisch die vorrangig relevanten, angreifenden Kräfte bzw. Größen, nämlich im Einzelnen die resultierende Kraft R1 vor der Rolle 10, die in Richtung der Feder wirkt, die resultierende Kraft R2 hinter der Rolle in Richtung, die in Richtung der Feder wirkt, die der Okklusion entgegenwirkende Kraft F, die die Rückstellkraft F des Materials des Schlauches 20 ist, das Drehmoment M und den Prefilterdruck P.

Insbesondere die Rückstellkraft des Schlauchmaterials ändert sich im Verlauf der Behandlung stark.

Im Graphen gemäß Figur 2 sind die Kraft-/Weg-Kennlinien einiger Schlauchmaterialien dargestellt, nämlich von PVC und PP. Ab dem Weg von ca. 8 mm sind die Schläuche nahezu vollständig okkludiert. Der Schlauchdurchmesser beträgt hier 12 mm und die Wandstärke 2 mm.

Um die Schläuche vollständig zu okkludieren ist hier eine Kraft im Bereich von 40 N notwendig. Eine vollständige Okklusion des Schlauches wird meist erst oberhalb dieser Kraft erreicht.

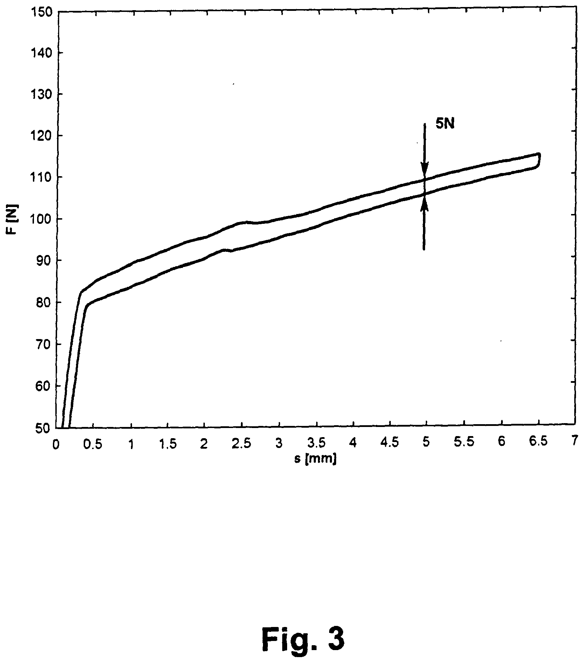

Betrachtet man die Federkennlinie (Hysteresemessung bei Umgebungstemperatur) eines "normalen" Rotors gemäß Figur 3, ist erkennbar, dass die Rückstellkraft des Schlauches von ca. 40 N etwa halb so groß ist wie die Federkraft des Rotors im Arbeitspunkt (ca. 90 N) und damit einen wesentlichen Einfluss auf den Druck hat, ab dem keine vollständige Okklusion mehr gegeben ist.

Während der Dialyse wird der Prefilterdruckverlauf kontinuierlich erfasst. Dieser kann als Kurvenverlauf analysiert oder zur Erhöhung der Toleranz gegenüber Änderungen im Pumpschlauchmaterial, Medium, etc. mit der Winkelposition des Blutpumpenrotors abgeglichen werden.

Wandert das Maximum des Druckverlaufs zu weit "nach links" (vgl. Figur 4), bzw. steigt der zeitliche Abstand zwischen Maximum und Minimum deutlich an, so hebt die Rolle vorzeitig ab und es besteht die Gefahr der Blutschädigung. Eine Reduzierung der Förderrate zur Wiederherstellung einer vollständigen Okklusion oder eine frühzeitige Alarmreaktion kann eingeleitet werden.

In Figur 4 wurden die Druckverläufe, bezogen auf die Rotorposition bei verschiedenen Gegendrücken aufgetragen. Dazu wurde ein Rotor mit zwei gegenüberliegenden Rollen mit verringerter Federkraft eingesetzt und der Fluß hinter der Pumpe ist ungleich 0 ml/min. Bei dem Rotor der Schlauchrollenpumpe, mit der das Diagramm gemäß Figur 4 ermittelt wurde, handelt es sich um einen Rotor mit zwei gegenüberliegenden Rollen. Beispielsweise bei einer Winkelstellung von α = 0° und α = 180° ist noch mit zumindest einer der Rollen voll okkludiert, wobei diese Rolle in Folge der weiteren Drehbewegung das Pumpenbett über die Auslaufschräge verlässt.

Grundsätzlich ist es aber möglich, die Schlauchrollenpumpe beliebig, insbesondere hinsichtlich Anzahl der Rollen und Pumpenbettgestaltung auszuführen.

Zu erkennen ist, dass mit zunehmendem Gegendruck, hier ab ca. 700 mbar, das Maximum des Prefilterdruckverlaufs "nach links" wandert, d. h. während der Rotordrehung zu einem früheren Zeitpunkt bzw. früheren Winkelposition α_{MAX} auftritt. Die Winkelposition α_{MIN} des Minimums hingegen bleibt praktisch unverändert.

Die oberste Kurve dP1 zeigt somit einen nahezu vollständig abgehobenen Blutpumpenrotor und unterscheidet sich deutlich von den übrigen Kurven.

drehung zu einem früheren Zeitpunkt bzw. früheren Winkelposition α_{MAX} auftritt. Die Winkelposition α_{MIN} des Minimums hingegen bleibt praktisch unverändert.

Die oberste Kurve dP1 zeigt somit einen nahezu vollständig abgehobenen Blutpumpenrotor und unterscheidet sich deutlich von den übrigen Kurven.

## Patentansprüche

1. Vorrichtung mit wenigstens einer Schlauchrollenpumpe, wobei die Schlauchrollenpumpe wenigstens ein Pumpenbett und wenigstens einen in das Pumpenbett eingelegten oder einlegbaren Schlauch (20) oder Schlauchteil aufweist, und mit wenigstens einem stromabwärts der Schlauchrollenpumpe angeordneten Druckerfassungsmittel, das so konfiguriert ist, dass es den zeitlichen Verlauf des Druckes eines mittels der Schlauchrollenpumpe geförderten Fluids ermittelt, und mit wenigstens einem Überwachungsmittel, das so konfiguriert ist, dass es die Okklusion des in das Pumpenbett eingelegten oder einlegbaren Schlauches (20) oder Schlauchteils anhand der Zeit- und/oder der Winkeldifferenz zwischen wenigstens einem Maximum und wenigstens einem Minimum des ermittelten zeitlichen Verlaufs des Druckes und/oder anhand eines lokalen Minimums des ermittelten zeitlichen Verlaufes des Druckes überwacht,
**dadurch gekennzeichnet,**
**dass** die Vorrichtung wenigstens ein Steuer- und/oder Regelungsmittel aufweist und das Steuer- und/oder Regelungsmittel so konfiguriert ist, dass es in einem Fall, in dem eine verringerte Okklusion erkannt wird, die Förderrate zur oder bis zur Wiederherstellung einer vollständigen Okklusion automatisch oder halbautomatisch reduziert.

2. Vorrichtung nach Anspruch 1, **dadurch gekennzeichnet, dass** das Druckerfassungsmittel stromabwärts der Schlauchrollenpumpe und stromaufwärts eines Filters, insbesondere eines Dialysators, angeordnet ist, und der Druckverlauf in diesem Bereich ein Prefilterdruckverlauf ist, wobei der stromabwärts der Schlauchrollenpumpe erfasste zeitliche Verlauf des Druckes der Prefilterdruckverlauf ist und das Druckerfassungsmittel so konfiguriert ist, dass es den Prefilterdruckverlauf kontinuierlich erfasst.

3. Vorrichtung nach einem der vorhergehenden Ansprüche, **dadurch gekennzeichnet, dass** das Überwachungsmittel so konfiguriert ist, dass es anhand eines Ansteigens des zeitlichen Abstandes zwischen Maximum und Minimum des zeitlichen Verlaufs des Druckes eine verringerte Okklusion erkennt, und insbesondere ein vorzeitiges Abheben einer Rolle (10) der Schlauchrollenpumpe erkennt.

4. Vorrichtung nach einem der vorhergehenden Ansprüche, **dadurch gekennzeichnet, dass** das Überwachungsmittel so konfiguriert ist, dass es anhand des Auftretens eines lokalen Minimums des ermittelten zeitlichen Verlaufes des Druckes eine verringerte Okklusion erkennt, und insbesondere ein vorzeitiges Abheben einer Rolle (10) der Schlauchrollenpumpe erkennt.

5. Vorrichtung nach Anspruch 3 oder 4, **dadurch gekennzeichnet, dass** die Vorrichtung wenigstens ein Warnmittel aufweist und dass das Warnmittel so konfiguriert ist, dass es in einem Fall, in dem eine verringerte Okklusion erkannt wird, wenigstens eine Warnmeldung abgibt.

6. Vorrichtung nach einem der vorhergehenden Ansprüche, **dadurch gekennzeichnet, dass** das Überwachungsmittel so konfiguriert ist, dass es die Überwachung des zeitlichen Verlaufs des Druckes zusammen mit einem Abgleich der Winkelposition des Schlauchrollenrotors der Schlauchrollenpumpe durchführt.

7. Vorrichtung nach einem der vorhergehenden Ansprüche, **dadurch gekennzeichnet, dass** die Vorrichtung Bestandteil einer Blutbehandlungsvorrichtung ist und/oder mit einer Blutbehandlungsvorrichtung in Verbindung steht und/oder eine Blutbehandlungsvorrichtung ist und/oder umfasst.

8. Vorrichtung nach einem der vorhergehenden Ansprüche, **dadurch gekennzeichnet, dass** die Vorrichtung Mittel aufweist, die so konfiguriert sind, dass sie die Winkelposition des Rotors der Schlauchrollenpumpe erfassen.

9. Blutbehandlungsvorrichtung, insbesondere Hämodialysemaschine, mit wenigstens einer Vorrichtung nach einem der vorhergehenden Ansprüche.

## Claims

1. An apparatus having at least one hose roller pump, wherein the hose roller pump comprises at least one pump bed and at least one hose (20) or hose part inserted or insertable into the pump bed, and having at least one pressure detection means which is arranged downstream of the hose roller pump and which is configured such that it determines the time profile of the pressure of a fluid conveyed by means of the hose roller pump, and having at least one monitoring means which is configured in such a way that is monitors the occlusion of the hose (20) or hose part inserted or insertable into the pump bed based upon the time difference and/or to the angular difference between at least one maximum and at least one minimum of the determined time profile of the pressure and/or based upon a local minimum of the determined time profile of the pressure,
**characterized in that**
the apparatus comprises at least one control and/or regulation means and the control and/or regulation means is configured in such a way that, in a case in which a reduced occlusion is recognized, it automatically or semi-automatically reduces the conveying rate for or up to the restoration of a complete occlusion.

2. An apparatus in accordance with claim 1, **characterized in that** the pressure detection means is arranged downstream of the hose roller pump and upstream of a filter, in particular of a dialyzer, and the pressure profile in this region is a prefilter pressure profile, with the time profile of the pressure detected downstream of the hose roller pump being the prefilter pressure profile, and the pressure detection means is configured in such a way that it detects the prefilter pressure profile continuously.

3. An apparatus in accordance with any one of the preceding claims, **characterized in that** the monitoring means is configured in such a way that it recognizes a reduced occlusion, in particular a premature lifting off of a roller (10) of the hose roller pump based upon an increase in the time interval between the maximum and minimum of the time profile of the pressure.

4. An apparatus in accordance with one of the preceding claims, **characterized in that** the monitoring means is configured in such a way that is recognizes a reduced occlusion, in particular a premature lifting off of a roller (10) of the hose roller pump based upon the occurrence of a local minimum of the determined time profile of the pressure.

5. An apparatus in accordance with claim 3 or 4, **characterized in that** the apparatus comprises at least one warning means and **in that** the warning means is configures such that it outputs at least one warning in a case where a reduced occlusion is recognized.

6. An apparatus in accordance with one of the preceding claims, **characterized in that** the monitoring means is configured in such a way that it performs the monitoring of the time profile of the pressure together with a comparison of the angular position of the hose roller rotor of the hose roller pump.

7. An apparatus in accordance with one of the preceding claims, **characterized in that** the apparatus is part of a blood treatment apparatus and/or is in communication with a blood treatment apparatus and/or is and/or includes a blood treatment apparatus.

8. An apparatus in accordance with one of the preceding claims, **characterized in that** the apparatus comprises means which are configured in such a way that they detect the angular position of the rotor of the hose roller pump.

9. A blood treatment apparatus, in particular a hemodialysis machine, having at least one apparatus in accordance with one of the preceding claims.

## Revendications

1. Dispositif doté d'au moins une pompe péristaltique, ladite pompe péristaltique présentant au moins un lit de pompe et au moins un tuyau flexible (20) ou une partie de flexible placé(e) ou pouvant être placé(e) dans le lit de pompe et d'au moins un moyen de détection de pression disposé en aval de la pompe péristaltique qui est configuré de sorte à déterminer la courbe de variation dans le temps de la pression d'un fluide transporté au moyen de la pompe péristaltique, et d'au moins un moyen de surveillance configuré de sorte à surveiller l'occlusion du flexible (20) ou de la partie de flexible placé(e) ou pouvant être placé(e) dans le lit de pompe à l'aide de la différence de temps et/ou d'angle entre au moins un maximum et au moins un minimum de la courbe de variation dans le temps de la pression déterminée et/ou à l'aide d'un minimum local de la courbe de variation dans le temps de la pression déterminée,
**caractérisé en ce que**
le dispositif présente au moins un moyen de commande et/ou de régulation, ledit moyen de commande et/ou de régulation étant configuré de telle sorte que, lorsqu'une occlusion faible est détectée, le débit d'alimentation est réduit de manière automatique ou semi-automatique pour ou jusqu'au rétablissement d'une occlusion complète.

2. Dispositif selon la revendication 1, **caractérisé en ce que** le moyen de détection de pression est disposé en aval de la pompe péristaltique et en amont d'un filtre, en particulier d'un générateur de dialyse, et **en ce que** la courbe de variation de la pression dans cette plage est une courbe de variation de la pression du préfiltre, la courbe de variation dans le temps de la pression enregistrée en aval de la pompe péristaltique étant la courbe de variation de la pression du préfiltre et le moyen de détection de pression étant configuré de sorte à enregistrer la courbe de variation dans le temps de la pression du préfiltre de manière continue.

3. Dispositif selon l'une quelconque des revendications précédentes, **caractérisé en ce que** le moyen de surveillance est configuré de sorte à déceler une occlusion faible grâce à une augmentation de la différence dans le temps entre un maximum et un minimum de la courbe de variation dans le temps de la pression, et en particulier un soulèvement prématuré d'un rouleau (10) de la pompe péristaltique.

4. Dispositif selon l'une quelconque des revendications précédentes, **caractérisé en ce que** le moyen de surveillance est configuré de sorte à déceler une occlusion faible grâce à l'apparition d'un minimum local de la courbe de variation dans le temps de la pression déterminée, et en particulier un soulèvement prématuré d'un rouleau (10) de la pompe péristaltique.

5. Dispositif selon la revendication 3 ou 4, **caractérisé en ce que** ledit dispositif est doté d'au moins un moyen d'avertissement et **en ce que** ledit moyen d'avertissement est configuré de sorte à émettre au moins un message d'avertissement lorsqu'une occlusion faible est détectée.

6. Dispositif selon l'une quelconque des revendications précédentes, **caractérisé en ce que** le moyen de surveillance est configuré de sorte à réaliser la surveillance de la courbe de variation dans le temps de la pression avec une comparaison de la position angulaire du rotor à rouleaux de la pompe péristaltique.

7. Dispositif selon l'une quelconque des revendications précédentes, **caractérisé en ce que** ledit dispositif fait partie d'un dispositif de traitement du sang et/ou est an communication avec un dispositif de traitement du sang et/ou est un dispositif de traitement du sang et/ou comprend un dispositif de traitement du sang.

8. Dispositif selon l'une quelconque des revendications précédentes, **caractérisé en ce que** ledit dispositif présente de moyens qui sont configurés de sorte à enregistrer la position angulaire du rotor de la pompe péristaltique.

9. Dispositif de traitement du sang, en particulier une machine d'hémodialyse, comportant au moins un dispositif selon l'une quelconque des revendications précédentes.
